# EUROPEAN PATENT SPECIFICATION

(11) **EP 3 098 867 B1**
(45) Date of publication and mention of the grant of the patent: **21.11.2018**
(21) Application number: 16171286.4
(22) Date of filing: 25.05.2016
(51) Int. Cl.: H01L 41/09

(54) **PIEZOELECTRONIC DEVICE, PROBE, ELECTRONIC APPARATUS, AND ULTRASONIC IMAGING APPARATUS**
PIEZOELEKTRISCHE VORRICHTUNG, SONDE, ELEKTRONISCHE VORRICHTUNG UND ULTRASCHALLABBILDUNGSVORRICHTUNG
DISPOSITIF PIEZOÉLECTRONIQUE, SONDE, APPAREIL ÉLECTRONIQUE ET APPAREIL D'IMAGERIE PAR ULTRASONS

(30) Priority: 29.05.2015 JP 2015109530
(43) Date of publication of application: 30.11.2016
(73) Proprietor: Seiko Epson Corporation, Tokyo 160-8801 (JP)
(72) Inventor: MIYAZAWA, Hiromu, Nagano, 392-8502 (JP); ISSHIKI, Tetsuya, Nagano, 392-8502 (JP); ITO, Hiroshi, Nagano, 392-8502 (JP); YAMADA, Masayoshi, Nagano, 392-8502 (JP); TSURUNO, Jiro, Nagano, 392-8502 (JP); NAKAMURA, Tomoaki, Nagano, 392-8502 (JP)
(74) Representative: Hoffmann Eitle

(56) References cited:
- US-A1- 2001 022 487
- US-A1- 2003 234 835
- US-A1- 2011 018 944
- US-A1- 2012 043 485

## Description

### BACKGROUND

### 1. Technical Field

The present invention relates to a piezoelectric device and a probe, an electronic apparatus, and an ultrasonic imaging apparatus using the same.

### 2. Related Art

As disclosed in JP-A-2014-146722, a piezoelectric device includes a piezoelectric body interposed between first and second electrodes. In the piezoelectric body, (100) preferential orientation is established. According to such preferential orientation, the piezoelectric characteristics of the piezoelectric body are substantially enhanced.

As disclosed in JP-A-2002-271897, an ultrasonic transducer includes a piezoelectric device. In the piezoelectric device, first and second electrodes are disposed on the same surface of the piezoelectric body. In the piezoelectric body, (001) preferential orientation is established. In the same manner as in JP-A-2014-146722, a piezoelectric body with (100) preferential orientation is interposed between the first and second electrodes. However, in a case where the first and second electrodes are disposed on the same surface in the piezoelectric body, the (100) preferential orientation is not established in the piezoelectric body.

A piezoelectric device according to the preamble of claim 1 is known from US-A1-2001/022487. Similar devices are described in US-A1-2011/018944 and US-A1-2012/043485. US-A1-2003/0234835 also forms part of the prior art.

### SUMMARY

The object of the invention is to provide a piezoelectric device that establishes (100) preferential orientation in a piezoelectric body in a case where first and second electrodes are disposed on the same surface in the piezoelectric body.
(1) An aspect of the invention relates to a piezoelectric device including: an elastic layer that forms an insulating surface region at least partially and has an amorphous structure or random orientation at least in the surface region; a piezoelectric body that is provided on the elastic layer, has a first surface in contact with the elastic layer and a second surface on an opposite side to the first surface, and is (100) preferentially oriented in an orientation region corresponding to the surface region in a plan view; a first electrode electrically connected to the piezoelectric body; and a second electrode electrically connected to the piezoelectric body. Preferably or alternatively, the first electrode is provided on the second surface of the piezoelectric body and/or the second electrode is provided on the second surface of the piezoelectric body. A gap is formed between the first and second electrodes corresponding to the orientation region in the plan view, and a groove is formed on the second surface of the piezoelectric body in the gap.
   If the elastic layer vibrates due to the ultrasonic wave from the outside, the piezoelectric body is distorted on the elastic layer. The strain of the piezoelectric body generates an electric potential in the piezoelectric body. Since the (100) preferential orientation is established in the orientation region of the piezoelectric body, a relatively large electric potential is generated for the strain of the piezoelectric body. Such an electric potential generated by the piezoelectric response is detected as an electric potential difference between the first and second electrodes. In particular, since the bottom surface of the orientation region is in contact with the insulating surface region, the formation of a parasitic capacitance connecting the electrodes to each other is avoided. Accordingly, good receiving sensitivity can be secured. If a conductive material is present on the bottom surface of the orientation region, a parasitic capacitance connecting the electrodes to each other is formed. Therefore, the receiving sensitivity is lowered. In order to obtain high receiving sensitivity, it is preferable that electrical flux lines extend directly to the second electrode through the piezoelectric body from the first electrode.
(2) The piezoelectric body may be formed of a transition metal oxide having a perovskite structure. In this case, the piezoelectric body shows good piezoelectric characteristics.
(3) The first surface of the piezoelectric body may be formed as an orientation control layer that is formed of a transition metal oxide having a perovskite structure. In this case, the piezoelectric body shows good piezoelectric characteristics.
(4) The first surface of the piezoelectric body may be formed as an orientation control layer that is formed of a transition metal oxide having a perovskite structure that contains bismuth in an A site and iron and titanium in a B site. Such a transition metal oxide establishes the (100) preferential orientation at the time of lamination on the elastic layer with an amorphous structure or random orientation. Here, since the orientation control layer has an insulation property, the formation of a parasitic capacitance connecting the electrodes to each other can be avoided. In addition, other rare earths, such as lanthanum (La), neodymium (Nd), samarium (Sm), and praseodymium (Pr), may be included in the A site. In addition, lead (Pb) may be further included in the A site.
(5) The resistance value of the orientation control layer may be equal to or greater than 10⁶ Ωcm. If the resistance value satisfying this condition is used, the formation of a parasitic capacitance connecting the electrodes to each other is reliably avoided.
(6) The orientation control layer is (100) preferentially oriented.
(7) The piezoelectric body may include a Pb(ZrTi)O₃ layer laminated on the orientation control layer. The Pb(ZrTi)O₃ layer establishes the (100) preferential orientation by being laminated on the orientation control layer.
(8) Pb may infiltrate into the orientation control layer. At the time of lamination of the Pb(ZrTi)O₃ layer that is a piezoelectric body, the Pb(ZrTi)O₃ layer may be heated to 600°C to 750°C. In this case, Pb contained in the Pb(ZrTi)O₃ layer infiltrates into the orientation control layer to be stabilized.
(9) The orientation control layer may be a continuous layer. If the orientation control layer is a continuous layer, the (100) preferential orientation can be reliably established in the Pb(ZrTi)O₃ layer.
(10) The orientation control layer may be a discontinuous layer. Even if the orientation control layer is a discontinuous layer, the (100) preferential orientation can be established in the Pb(ZrTi)O₃ layer.
(11) The groove is formed on the second surface of the piezoelectric body in the gap. By forming the groove, the in-plane strain of the elastic layer due to sound pressure is concentrated on the gap. Therefore, a large voltage is generated between the first and second electrodes.
(12) The piezoelectric device may be used by being built into a probe. In this case, the probe may include the piezoelectric device and a housing that supports the piezoelectric device.
(13) The piezoelectric device may be used by being built into an electronic apparatus. In this case, an electronic apparatus may include the piezoelectric device; and a processing circuit that is connected to the piezoelectric device and processes an output of the piezoelectric device.
(14) The piezoelectric device may be used by being built into an ultrasonic imaging apparatus. In this case, the ultrasonic imaging apparatus may include: the piezoelectric device; a processing circuit that is connected to the piezoelectric device and processes an output of the piezoelectric device to generate an image; and a display device that displays the image.

### BRIEF DESCRIPTION OF THE DRAWINGS

The invention will be described with reference to the accompanying drawings, wherein like numbers reference like elements.
Fig. 1 is an external view schematically showing a specific example of an electronic apparatus according to an embodiment, that is, an ultrasonic diagnostic apparatus.
Fig. 2 is an enlarged plan view schematically showing the configuration of an ultrasonic device unit.
Fig. 3 is an enlarged partial plan view of the ultrasonic device unit that schematically shows a region of a transmission array.
Fig. 4 is an enlarged vertical sectional view taken along the line A-A of Fig. 1.
Fig. 5 is an enlarged partial plan view of the ultrasonic device unit that schematically shows a first piezoelectric device according to a first embodiment, not part of the invention.
Fig. 6 is a vertical sectional view taken along the line B-B of Fig. 5.
Figs. 7A to 7D are side sectional views showing a form of BiFeTiO₃ that is an orientation control layer.
Fig. 8A is a diagram showing the electric potential distribution in a piezoelectric body in a gap between electrodes, which is obtained by calculation using a finite element method (FEM), in a case where the insulation of the orientation control layer is maintained, and Fig. 8B is a diagram showing the electric potential distribution in the piezoelectric body in the gap between electrodes, which is obtained by calculation using the finite element method (FEM), in a case where the orientation control layer is metal.
Fig. 9 is a diagram showing the relationship between a piezoelectric constant and the orientation direction of PZT calculated by electronic state calculation based on the first principle of solid.
Fig. 10A is an X-ray diffraction pattern of a piezoelectric layer formed on BiFeTiO₃ that is an orientation control layer in a case where the piezoelectric layer is PZT, and Fig. 10B is an X-ray diffraction pattern of the piezoelectric layer formed on BiFeTiO₃ that is an orientation control layer in a case where the piezoelectric layer is BFM-BT.
Fig. 11 corresponds to Fig. 5, and is an enlarged partial plan view of an ultrasonic device unit that schematically shows a first piezoelectric device according to a second embodiment which is in accordance with the invention.

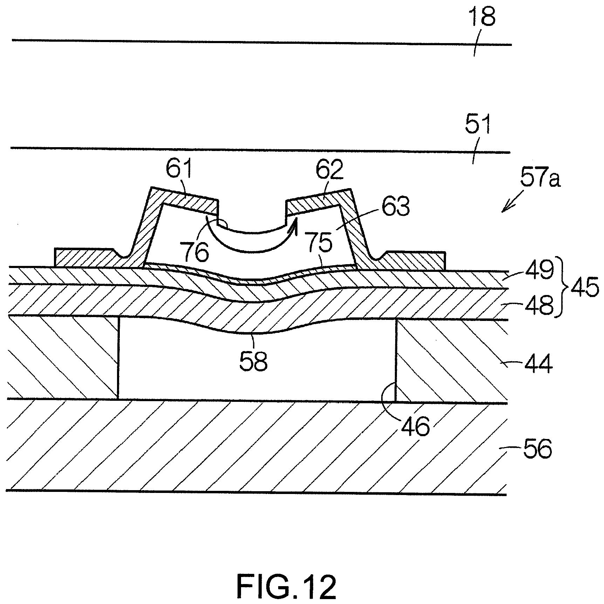

Fig. 12 is a vertical sectional view at the time of operation taken along the line C-C of Fig. 11.
Fig. 13 is an enlarged sectional view of a substrate in a manufacturing process of the first piezoelectric device.
Fig. 14 is an enlarged sectional view of a substrate schematically showing a piezoelectric body and an underlying conductive layer in the manufacturing process of the first piezoelectric device.
Fig. 15 is an enlarged sectional view of a substrate schematically showing first and second electrodes in the manufacturing process of the first piezoelectric device.

### DESCRIPTION OF EXEMPLARY EMBODIMENTS

Hereinafter, an embodiment not part of the invention will be described with reference to the accompanying diagrams. In addition, the present embodiment to be described below does not unduly limit the content of the invention as defined in the appended claims, and all elements described in the present embodiment are not necessarily indispensable as solving means of the invention.

### 1 Overall configuration of an ultrasonic diagnostic apparatus

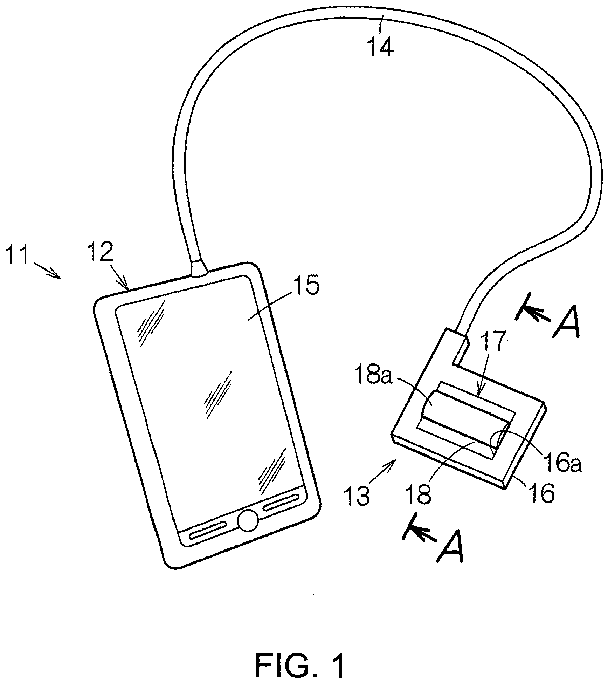

Fig. 1 schematically shows the configuration of a specific example of an electronic apparatus according to an embodiment not forming part of the invention, that is, the configuration of an ultrasonic diagnostic apparatus (ultrasonic imaging apparatus) 11. The ultrasonic diagnostic apparatus 11 includes a device terminal (processing unit) 12 and an ultrasonic probe (probe) 13. The device terminal 12 and the ultrasonic probe 13 are connected to each other by a cable 14. The device terminal 12 and the ultrasonic probe 13 perform transmission and reception of electrical signals therebetween through the cable 14. A display panel (display device) 15 is built into the display device 12. The screen of the display panel 15 is exposed on the surface of the device terminal 12. In the device terminal 12, an image is generated on the basis of the ultrasonic wave detected by the ultrasonic probe 13. An imaged detection result is displayed on the screen of the display panel 15.

The ultrasonic probe 13 includes a housing 16. The ultrasonic device unit 17 is housed in the housing 16. The ultrasonic device unit 17 includes an acoustic lens 18. A partial cylindrical surface 18a is formed as the outer surface of the acoustic lens 18. The acoustic lens 18 is formed of, for example, silicone resin. The acoustic lens 18 has an acoustic impedance close to the acoustic impedance of the living body. A window hole 16a is defined in the housing 16. The acoustic lens 18 is disposed in the window hole 16a. The outer surface of the acoustic lens 18 is exposed on the surface of the housing 16. The ultrasonic device unit 17 outputs an ultrasonic wave from the surface and receives a reflected wave of the ultrasonic wave.

### 2 Configuration of the ultrasonic device unit

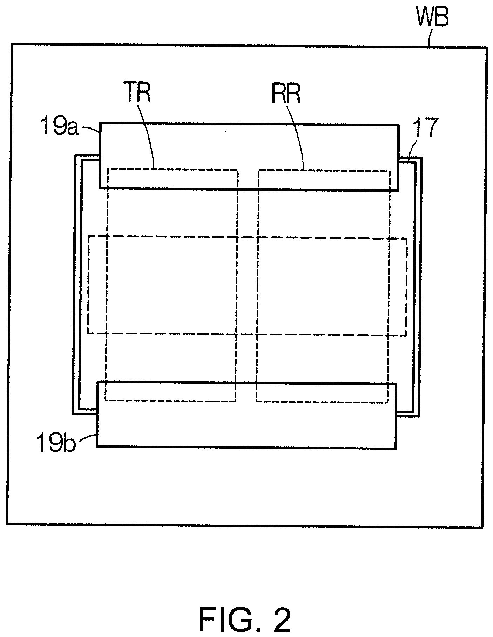

As shown in Fig. 2, the ultrasonic device unit 17 includes a wiring substrate WB. The ultrasonic device unit 17 is mounted on the wiring substrate WB. In such mounting, a hollow to receive the ultrasonic device unit 17 therein may be formed on the surface of the wiring substrate WB. The hollow may be recessed from the plane of the wiring substrate WB. The ultrasonic device unit 17 can be fixed to the wiring substrate WB with a resin material, for example.

A receiving array RR and a transmission array TR are formed in the ultrasonic device unit 17. As will be described later, the receiving array RR is formed as an array of first ultrasonic transducer elements (hereinafter, referred to as "first piezoelectric devices") that are disposed in the form of an array. As will be described later, the transmission array TR is formed as an array of second ultrasonic transducer elements (hereinafter, referred to as "second piezoelectric devices") that are disposed in the form of an array. The receiving array RR and the transmission array TR are electrically connected to a wiring pattern (not shown) on the wiring substrate through a first flexible printed wiring board (hereinafter, referred to as a "first wiring board") 19a and a second flexible printed wiring board (hereinafter, referred to as a "second wiring board") 19b. The wiring pattern is connected to a connector on the back surface of the wiring board WB. The cable 14 is formed by the wiring lines connected to the connector.

### 3 Configuration of the transmission array

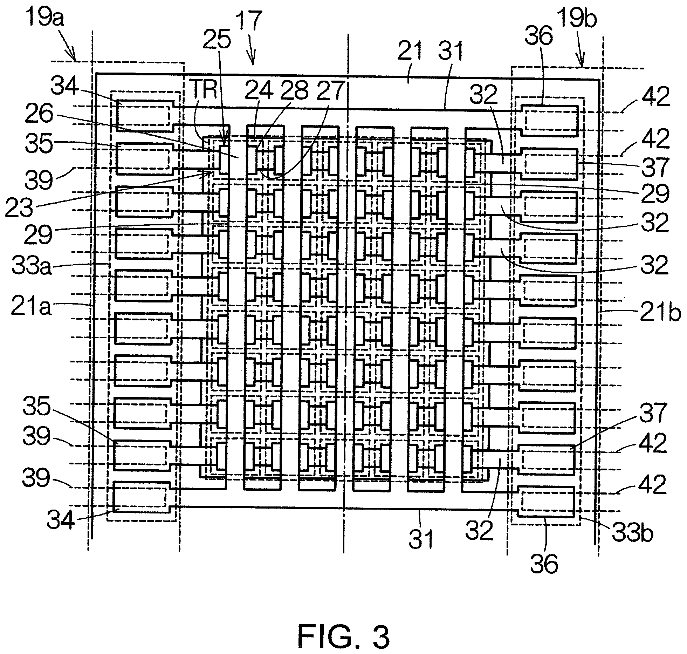

Fig. 3 schematically shows a plan view of the ultrasonic device unit 17 for a region of the transmission array TR. The ultrasonic device unit 17 includes a base 21. The transmission array TR is formed on the surface of the base 21. The array of the second piezoelectric devices 23 is formed in a matrix of rows and columns. In addition, in the array, a staggered arrangement may be established. In the staggered arrangement, a group of second piezoelectric devices 23 in the even-numbered column may be shifted by 1/2 of the row pitch from a group of second piezoelectric devices 23 in the odd-numbered column. The number of elements in one of the odd-numbered column and the even-numbered column may be one smaller than the number of elements in the other column.

Each of the second piezoelectric devices 23 includes a vibrating film 24. In Fig. 3, the contour of the vibrating film 24 is drawn by a dotted line in a plan view from a direction perpendicular to the surface of the vibrating film 24 (in a plan view from the thickness direction of the substrate; hereinafter, simply referred to as "in a plan view"). A vibrator 25 is formed on the vibrating film 24. The vibrator 25 includes an upper electrode 26, a lower electrode 27, and a piezoelectric body 28. For each second piezoelectric device 23, the lower electrode 27 is disposed on the vibrating film 24, the piezoelectric body 28 is disposed on the lower electrode 27, and the upper electrode 26 is disposed on the piezoelectric body 28. These overlap each other in order of the lower electrode 27, the piezoelectric body 28, and the upper electrode 26. In this manner, the piezoelectric body 28 is interposed between the upper electrode 26 and the lower electrode 27.

A plurality of first conductors 29 are formed on the surface of the base 21. The first conductors 29 extend in parallel in the row direction of the array. One first conductor 29 is assigned to the second piezoelectric devices 23 in one row. The first conductor 29 forms the upper electrode 26 in each second piezoelectric device 23. Both ends of the first conductor 29 are connected to a pair of lead-out wiring lines 31. The first conductor 29 can be formed of, for example, iridium (Ir). However, other conductive materials may be used for the first conductor 29.

A plurality of second conductors 32 are formed on the surface of the base 21. The second conductors 32 extend in parallel in the column direction of the array. One second conductor 32 is assigned to the second piezoelectric devices 23 in one column. The second conductor 32 forms the lower electrode 27 in each second piezoelectric device 23. For the second conductor 32, for example, a laminated film of titanium (Ti), iridium (Ir), platinum (Pt), and titanium (Ti) can be used. However, other conductive materials may be used for the second conductor 32.

The supply of power to the second piezoelectric device 23 is switched for each column. A linear scan or a sector scan is realized according to such switching of power supply. Since the second piezoelectric devices 23 in one column output ultrasonic waves at the same time, the number of devices in a column, that is, the number of rows of the array, can be determined according to the output level of the ultrasonic wave. For example, the number of rows may be set to about 10 to 15. In Fig. 3, five rows are drawn, and other rows are omitted. The number of columns of the array can be determined according to the spread of the scan range. For example, the number of columns may be set to 128 or 256. In Fig. 3, eight columns are drawn, and other columns are omitted. The roles of the upper electrode 26 and the lower electrode 27 may be exchanged. That is, a lower electrode may be connected in common to the second piezoelectric devices 23 in the entire matrix, and an upper electrode may be connected to the second piezoelectric devices 23 in common to respective columns of the array.

The contour of the base 21 has first and second sides 21a and 21b that are a pair of parallel straight lines facing each other. A first terminal array 33a of one line is disposed between the first side 21a and the contour of the transmission array TR. A second terminal array 33b of one line is disposed between the second side 21b and the contour of the transmission array TR. The first terminal array 33a can form one line in parallel to the first side 21a. The second terminal array 33b can form one line in parallel to the second side 21b. The first terminal array 33a is formed by a pair of upper electrode terminals 34 and a plurality of lower electrode terminals 35. Similarly, the second terminal array 33b is formed by a pair of upper electrode terminals 36 and a plurality of lower electrode terminals 37. The upper electrode terminals 34 and 36 are connected to the ends of one lead-out wiring lines 31. The lead-out wiring line 31 and the upper electrode terminals 34 and 36 may be formed plane-symmetrically on a vertical plane bisecting the transmission array TR. The lower electrode terminals 35 and 37 are connected to both ends of the one second conductor 32. The second conductor 32 and the lower electrode terminals 35 and 37 may be formed plane-symmetrically on the vertical plane bisecting the transmission array TR. Here, the contour of the base 21 is formed in a rectangular shape. The contour of the base 21 may be a square, or may be a trapezoid.

The first wiring board 19a is connected to the base 21. The first wiring board 19a covers the first terminal array 33a. At one end of the first wiring board 19a, a conductive line, that is, a first signal line 39 is formed corresponding to each of the upper electrode terminal 34 and the lower electrode terminal 35. Each first signal line 39 is separately bonded to the upper electrode terminal 34 and the lower electrode terminal 35 so as to face each other. Similarly, the second wiring board 19b is connected to the base 21. The second wiring board 19b covers the second terminal array 33b. At one end of the second wiring board 19b, a conductive line, that is, a second signal line 42 is formed corresponding to each of the upper electrode terminal 36 and the lower electrode terminal 37. Each second signal line 42 is separately bonded to the upper electrode terminal 36 and the lower electrode terminal 37 so as to face each other.

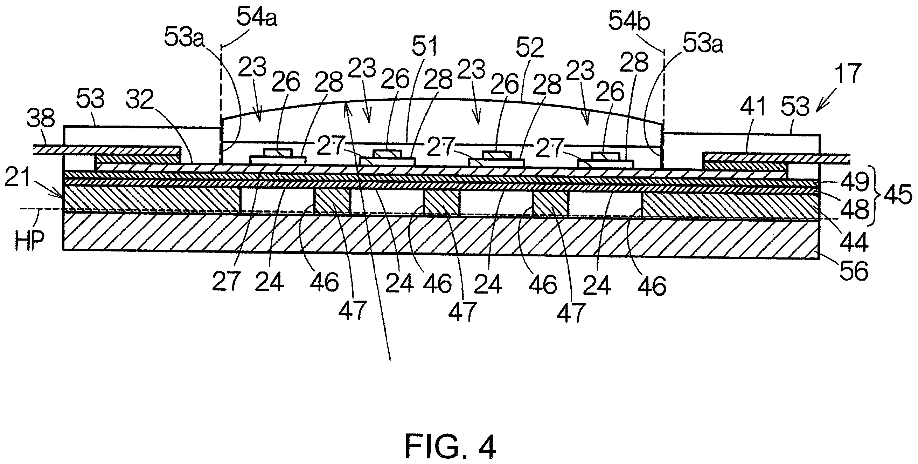

As shown in Fig. 4, the base 21 includes a substrate 44 and a coat layer (elastic layer) 45. The coat layer 45 is formed on the entire one surface of the substrate 44. On the substrate 44, an opening 46 is formed in each second piezoelectric device 23. The opening 46 is disposed in the form of an array on the substrate 44. The contour of a region where the opening 46 is disposed corresponds to the contour of the transmission array TR. A partition wall 47 is provided between two adjacent openings 46. The adjacent openings 46 are partitioned by the partition wall 47. The substrate 44 may be formed as a silicon substrate, for example.

The coat layer 45 is formed by a silicon oxide (SiO₂) layer 48 laminated on the surface of the substrate 44 and a zirconium oxide (ZrO₂) layer 49 laminated on the surface of the silicon oxide layer 48. The coat layer 45 is in contact with the opening 46. In this manner, corresponding to the contour of the opening 46, a part of the coat layer 45 forms the vibrating film 24. Since the vibrating film 24 faces the opening 46 of the coat layer 45, the vibrating film 24 is a part that can perform film vibration in the thickness direction of the substrate 44. The thickness of the silicon oxide layer 48 can be determined based on the resonance frequency.

The lower electrode 27, the piezoelectric body 28, and the upper electrode 26 are laminated in order on the surface of the vibrating film 24. The piezoelectric body 28 can be formed of, for example, lead zirconate titanate (Pb(ZrTi)O₃). Other piezoelectric materials may be used for the piezoelectric body 28.

An acoustic matching layer 51 is laminated on the surface of the base 21. The acoustic matching layer 51 covers the element array 22. The thickness of the acoustic matching layer 51 is determined according to the resonance frequency of the vibrating film 24. For example, a silicone resin film can be used as the acoustic matching layer 51. The acoustic lens 18 is disposed on the acoustic matching layer 51. The acoustic lens 18 is in close contact with the surface of the acoustic matching layer 51. The acoustic lens 18 is bonded to the base 21 by the acoustic matching layer 51. The partial cylindrical surface 18a of the acoustic lens 18 has a generatrix parallel to the first conductor 29. The curvature of the partial cylindrical surface 18a is determined according to the focal position of ultrasonic waves transmitted from the second piezoelectric devices 23 in one column that are connected to one second conductor 33. The acoustic lens 18 is formed of, for example, silicone resin. The acoustic lens 18 has an acoustic impedance close to the acoustic impedance of the living body.

A protective film 53 is fixed to the base 21. The protective film 53 is formed of, for example, a material having a water-proof property, such as an epoxy resin. However, the protective film 53 may be formed of other resin materials. The protective film 53 is in contact with the acoustic lens 18 and the acoustic matching layer 51. Here, the acoustic lens 18 and the acoustic matching layer 51 are interposed between contact surfaces 53a of the protective film 53, which are located along two virtual planes 54a and 54b that spread in parallel to the generatrix of the acoustic lens 18 and are perpendicular to the base 21.

A backing material 56 is fixed to the back surface of the base 21. The back surface of the base 21 overlaps the surface of the backing material 56. The backing material 56 closes the opening 46 on the back surface of the ultrasonic device unit 17. The backing material 56 can include a rigid base, for example. Here, the partition wall 47 is bonded to the backing material 56. The backing material 56 is bonded to each partition wall 47 in at least one bonding region. In the bonding, an adhesive can be used.

### 4 Configuration of the receiving array

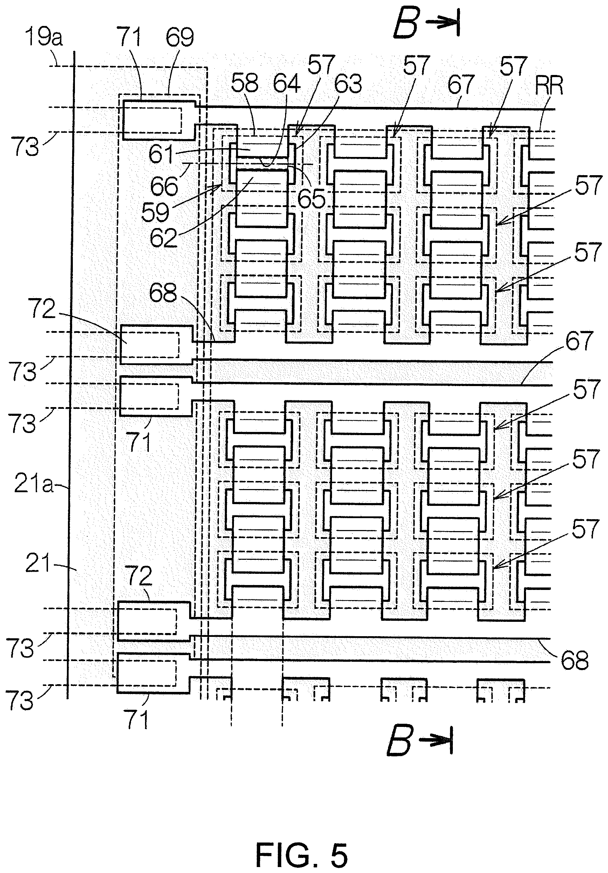

Fig. 5 schematically shows an enlarged partial plan view of the ultrasonic device unit 17 for a region of the receiving array RR. The receiving array RR is formed on the surface of the base 21. The array of the first piezoelectric devices 57 is formed in a matrix of rows and columns. The first piezoelectric device 57 according to the first embodiment includes a vibrating film (elastic layer) 58. In Fig. 5, the contour of the vibrating film 58 is drawn by a dotted line in a plan view from a viewing point perpendicular to the surface of the vibrating film 58. Similar to the vibrating film 24 described above, the vibrating film 58 is formed from the coat layer 45 on the surface of the substrate 44.

A vibrator 59 is formed on the vibrating film 58. The vibrator 59 includes a first electrode 61, a second electrode 62, and a piezoelectric body 63. The first and second electrodes 61 and 62 are disposed on the piezoelectric body 63. The second electrode 62 is disposed at a position separated from the first electrode 61 on the piezoelectric body 63. A gap 64 is formed between the first and second electrodes 61 and 62. On the surface of the vibrating film 58, a surface region 65 overlapping the gap 64 in a plan view is defined.

Here, the vibrating film 58 is formed in a rectangular shape (including a square) in a plan view. Similarly, the piezoelectric body 63 is formed in a rectangular shape in a plan view. The center of the vibrating film 58 and the center of the piezoelectric body 63 overlap each other in a plan view. The vibrator 59 is formed symmetrically with respect to a reference line 66. The reference line 66 extends in parallel to the long side of the rectangle to bisect the rectangle. At the ends of the first and second electrodes 61 and 62, a contour line is drawn in parallel to the reference line 66. Thus, the end of the first electrode 61 and the end of the second electrode 62 face each other at an equal distance with the reference line 66 interposed therebetween. The gap 64 extends along the reference line 66.

A plurality of third conductors 67 and a plurality of fourth conductor 68 are formed on the surface of the base 21. The third and fourth conductors 67 and 68 extend in parallel to each other in the column direction of the array. The third and fourth conductors 67 and 68 are alternately disposed. The first piezoelectric devices 57 of a plurality of columns are assigned for each pair of adjacent third and fourth conductors 67 and 68. Between the third and fourth conductors 67 and 68, the first piezoelectric devices 57 are connected in series to each other in each row. That is, in the first piezoelectric devices 57 adjacent to each other in a row, the first electrode 61 of one of the devices is connected to the second electrode 62 of the other device. The same materials can be used for the first electrode 61, the second electrode 62, the third conductor 67, and the fourth conductor 68. For example, a laminated film of titanium (Ti), iridium (Ir), platinum (Pt), and titanium (Ti) can be used for the first electrode 61, the second electrode 62, the third conductor 67, and the fourth conductor 68. However, other conductive materials may be used for the first electrode 61, the second electrode 62, the third conductor 67, and the fourth conductor 68.

Between the first side 21a of the base 21 and the contour of the receiving array RR, a third terminal array 69 of one line is disposed. The third terminal array 69 can form one line in parallel to the first side 21a. The third terminal array 69 includes a signal terminal 71 and a common terminal 72. The signal terminal 71 is connected to the third conductor 67. The common terminal 72 is connected to the fourth conductor 68. Here, the third terminal array 69 forms one line together with a first terminal array 38. The first wiring board 19a covers the first terminal array 33a and the third terminal array 69. At one end of the first wiring board 19a, a conductive line, that is, a third signal line 73 is formed corresponding to each of the signal terminal 71 and the common terminal 72. Each third signal line 73 is separately bonded to the signal terminal 71 and the common terminal 72 so as to face each other.

Similarly, between the second side 21b of the base 21 and the contour of the receiving array RR, a fourth terminal array (not shown) of one line may be disposed. The fourth terminal array may form one line together with a second terminal array 33b. The second wiring board 19b covers the second terminal array 33b and the fourth terminal array. At one end of the second wiring board 19b, a conductive line, that is, a fourth signal line is formed corresponding to each of the signal terminal and the common terminal. Each fourth signal line is separately bonded to the signal terminal and the common terminal so as to face each other.

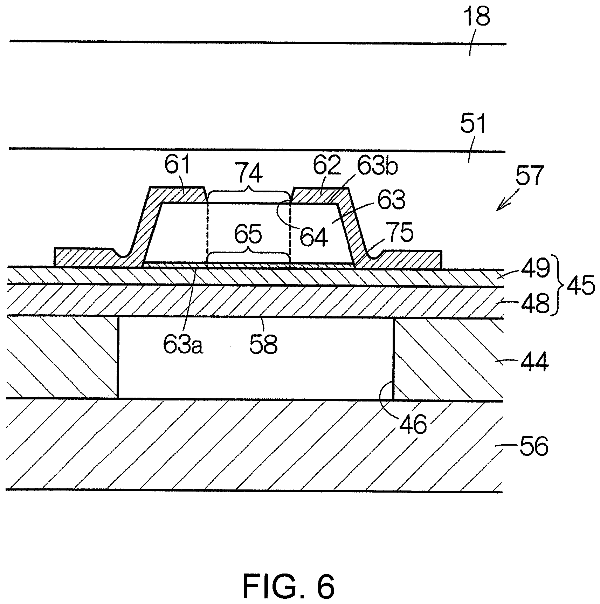

As shown in Fig. 6, on the substrate 21, the opening 46 is formed in each first piezoelectric device 57. The opening 46 is disposed in the form of an array on the substrate 44. The contour of a region where the opening 46 is disposed corresponds to the contour of the receiving array RR. Corresponding to the contour of the opening 46, a part of the coat layer 45 forms the vibrating film 58. The surface of the vibrating film 58 is formed by random orientation of the zirconium oxide layer 49. That is, the surface of the vibrating film 58 has an insulation property. On the surface of the vibrating film 58, the surface region 65 is partially partitioned.

The piezoelectric body 63 is fixed to the surface of the vibrating film 58. The piezoelectric body 63 has a first surface 63a in contact with the vibrating film 58 and a second surface 63b on the opposite side (back side) to the first surface 63a. The piezoelectric body 63 is formed of a piezoelectric material containing a composite oxide having a perovskite structure, that is, an ABO₃ type structure. The perovskite structure is a structure in which oxygen is 12-coordinated in the A site and is 6-coordinated in the B site to form an octahedron. The piezoelectric body 63 is formed of, for example, lead zirconate titanate (Pb(ZrTi)O₃). The piezoelectric body 63 may be formed of a transition metal oxide having a perovskite structure. For the piezoelectric body 63, for example, a material obtained by adding a metal oxide, such as a niobium oxide, a nickel oxide, or a magnesium oxide, to lead zirconate titanate may be used. Specifically, lead titanate (PbTiO₃), lead zirconate (PbZrO₃), lead lanthanum titanate ((PbLa)TiO₃), lead lanthanum zirconate titanate ((PbLa) (ZrTi)O₃), magnesium niobate zirconium titanate (Pb(ZrTi)(MgNb)O₃), and the like can be mentioned. In addition, as non-lead-based materials, bismuth ferrate (BiFeO₃), barium titanate (BaTiO₃), sodium potassium niobate ((KNa) (NbO₃)), potassium sodium lithium niobate ((KNaLi)(NbO₃)), potassium sodium lithium niobate tantalate ((KNaLi)(NbTa)O₃), bismuth potassium titanate ((Bi_{1/2}K_{1/2})TiO₃), bismuth sodium titanate ((Bi_{1/2}Na_{1/2})TiO₃), manganese bismuth (BiMnO₃), a composite oxide (x[(BiₓK₁₋ₓ)TiO₃] - (1-x)[BiFeO₃]) having a perovskite structure that contains bismuth, potassium, titanium, and iron, a composite oxide ((1-x)[BiFeO₃]-x[BaTiO₃]) having a perovskite structure that contains bismuth, iron, barium and titanium, a material ((1-x)[Bi(Fe_{1-y}M_{y})O₃]-x[BaTiO₃] (M is Mn, Co, or Cr)) obtained by adding a metal, such as manganese, cobalt, or chromium, to the composite oxide, and the like can be mentioned. Such a piezoelectric material having a perovskite structure is formed on an orientation control layer 75. Therefore, the piezoelectric material having a perovskite structure is preferentially oriented on the (100) plane according to the crystal orientation of the orientation control layer 75. Here, the preferential orientation refers to " (100) / [(100) + (110)] is 60% or more, preferably, 70% or more" when comparing the peak intensity derived from the (100) plane with the peak intensity derived from the (110) plane in an X-ray diffraction pattern. Having a large proportion of the peak of the (100) plane is effective in terms of displacement characteristics and durability of the piezoelectric body 63. It is preferable that (100) / [(100) + (110)] is 80% or more. More preferably, (100)/[(100)+(110)] is 90% or more. In the embodiment to be described later, it has been confirmed that (100)/[(100)+(110)] is 90% or more. From the viewpoint that the piezoelectric material has excellent displacement characteristics due to being preferentially oriented on the (100) plane, it is preferable that the piezoelectric material is rhombohedral or monoclinic. This, in the piezoelectric body 63, (100) preferential orientation is established. In the piezoelectric body 63, (100) preferential orientation is secured in at least an orientation region 74 corresponding to the surface region 65 in a plan view.

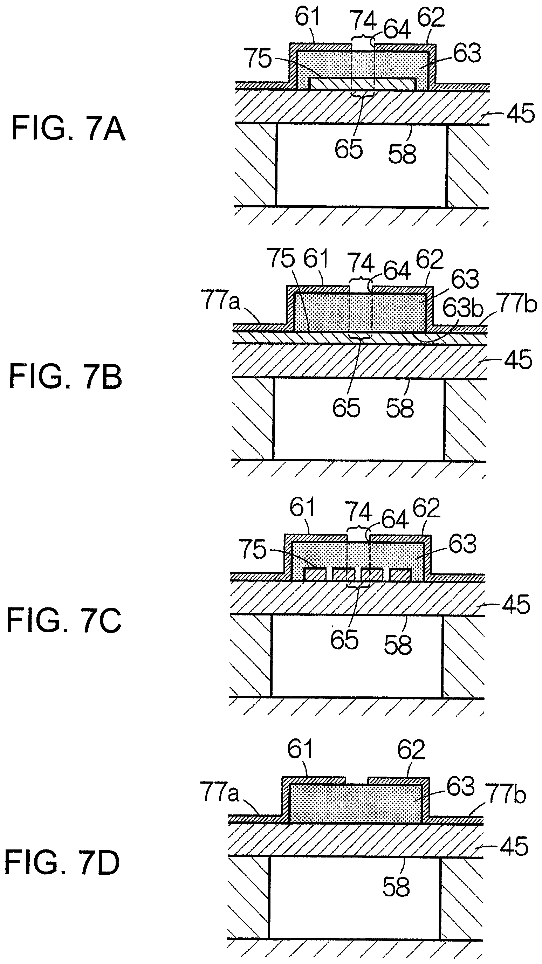

As shown in Fig. 7A, the orientation control layer 75 is (100) -oriented and is located on the bottom surface of the piezoelectric body 63, and determines the crystal orientation of the piezoelectric body 63. In order to realize a high potential difference by strain due to sound pressure, it is preferable that the gap 64 between the first and second electrodes 61 and 62 and the piezoelectric body 63 near the gap 64 are (100)-oriented. Therefore, at least the orientation control layer 75 should be disposed in the surface region 65 facing the gap 64. Here, the orientation control layer 75 is physically disconnected from the first and second electrodes 61 and 62. In this manner, it is possible to prevent dielectric breakdown through the orientation control layer 75 when an initializing voltage is applied between the first and second electrodes 61 and 62. Therefore, the reliability of the first piezoelectric device 57 is increased. In addition, as shown in Fig. 7B, the orientation control layer 75 may be present below the entire second surface 63b of the piezoelectric body 63 and lead-out wiring lines 77a and 77b of the first and second electrodes 61 and 62. In a case where the piezoelectric body is formed outside the region of the piezoelectric device, if (100) orientation is established in the piezoelectric body, resistance to cracks is increased. Fig. 7C shows the orientation control layer 75 present in a discontinuous island shape. Even if the orientation control layer 75 is present in a discontinuous island shape, the orientation control layer 75 is (100)-oriented. Therefore, the piezoelectric body 63 that grows on the orientation control layer 75 is (100) preferentially oriented with the island-shaped orientation control layer 75 as a growth core. The island shape described herein refers to a state in which the orientation control layer 75 is not present continuously with a uniform thickness. Accordingly, connections may be made partially between islands. In addition, the orientation control layer 75 may be formed in a stripe shape when viewed from above. Fig. 7D corresponds to a comparative example, and the orientation control layer 75 is not provided.

The orientation control layer 75 has a perovskite structure, is a composite oxide containing bismuth (Bi) in the A site and iron (Fe) and titanium (Ti) in the B site, and is self-orientated on the (100) plane. Specifically, in the A site of the ABO₃ type structure, oxygen is 12-coordinated. In addition, in the B site of the ABO₃ type structure, oxygen is 6-coordinated. As a result, an octahedron is formed. It is preferable that a composite oxide, which forms the orientation control layer 75, is basically formed of Bi of the A site and Fe and Ti of the B site. A preferred composition ratio is in the range of 40 ≤ x ≤ 60 in a case where the elemental ratio is expressed as Bi:Fe:Ti = 100:x: (100-x) . A composite oxide having such a composition ratio is self-oriented on the (100) plane without being influenced by an underlying layer, and functions as an orientation control layer for orienting a piezoelectric material having a perovskite structure, which is formed on the composite oxide, on the (100) plane. That is, as will be described in detail later, since the orientation control layer 75 is formed after the coat layer 45 is formed, the orientation control layer 75 is formed on a zirconium oxide layer 81. However, also on a silicon oxide layer 79, the orientation control layer 75 is self-oriented on the (100) plane. Accordingly, the piezoelectric body 63 formed subsequently can also be reliably preferentially oriented on the (100) plane.

Here, being self-oriented on the (100) plane refers to being oriented on the (100) plane by itself without being influenced by an underlying layer, and includes a case in which all the crystals are oriented on the (100) plane and a case in which most of the crystals (for example, 80% or more) are oriented on the (100) plane. A composite oxide obtained by replacing some of the elements of the A site or the B site with other elements may be used to the extent that such a function is not inhibited, and this is also included in the orientation control layer according to the invention. For example, elements other than Bi, such as Ba, Sr, and La, may be further present in the A site, and elements other than Fe and Ti, such as Zr and Nb, may be further present in the B site. In addition, as long as the function described above is realized, materials having a composition deviated from the composition (ABO₃) of stoichiometry due to deficiency or excess of an element (Bi, Fe, Ti, or O) are also included in the orientation control layer according to the invention. For example, as will be described later, it has been confirmed that a composite oxide, which contains Bi more than the stoichiometric ratio, is self-oriented on the (100) plane and functions as an orientation control layer.

### 5 Reason why the orientation control layer should be an insulator

The reason why the orientation control layer 75 should be an insulator is shown below using simulation. Simulation was performed using a finite element method (FEM) for realizing the piezoelectric effect. The vibrating film 58 and the piezoelectric body 63 were squares when viewed from above. The length of one side of a diaphragm formed by the vibrating film 58 was 40 µm, and the length of one side of the piezoelectric body 63 was 32 µm. In addition, the vibrating film 58 includes SiO₂ (1000 nm), ZrO₂ (400 nm), BFT (20 nm), and PZT (1350 nm) in order from below. BFT is the abbreviation of BiFeTiO₃ that forms the orientation control layer 75 according to the invention. The thickness of each of the first and second electrodes 61 and 62 was 50 nm. A line that passes through the center of the gap 64 between the first and second electrodes 61 and 62 and is parallel to each of the electrodes 61 and 62 is assumed to be the reference line 66. As the piezoelectric tensor and the stiffness tensor of PZT used in the simulation, a data set of PZT-5H was adopted. In this case, the principal axis of the tensor was set to a direction perpendicular to the reference line 66 (the same as a direction in which an initialization electric field was applied). The Young's moduli of SiO₂, ZrO₂, and BFT were 75 GPa, 190 GPa, and 200 GPa, respectively. The Young's moduli of the first and second electrodes 61 and 62 were 200 GPa. A 1 atmosphere was applied to the vibrating film 58 from above, and an electric potential V generated between the first and second electrodes 61 and 62 at that time was examined. In order to calculate the electric potential V, the first and second electrodes 61 and 62 were in the open condition. A width W1 between the first and second electrodes 61 and 62 was set to 5 [µm].

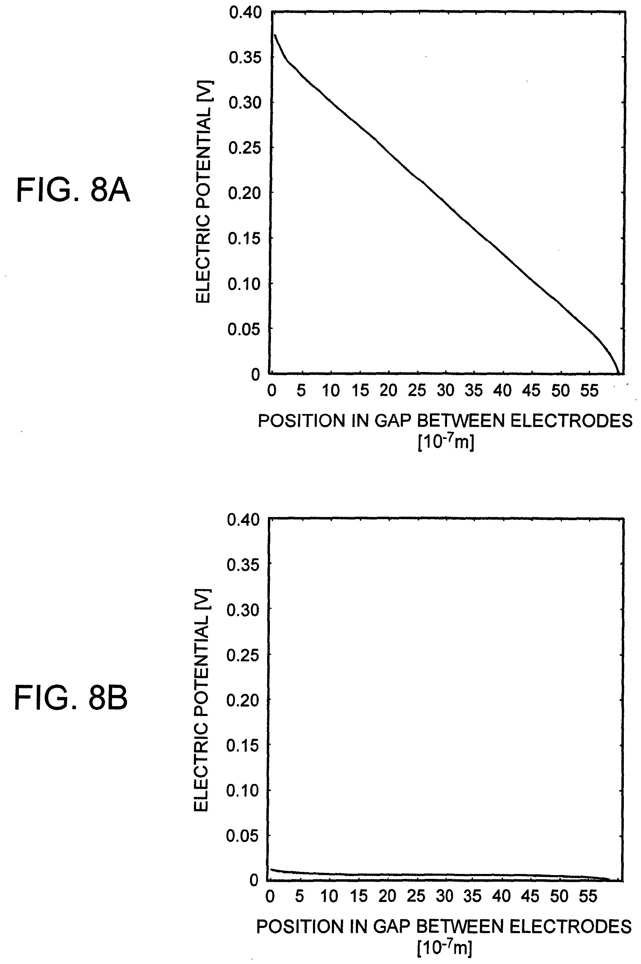

Fig. 8A is a diagram showing the electric potential V generated in a case where the orientation control layer 75 has an insulation property, Fig. 8B is a diagram showing the electric potential V generated in a case where a layer corresponding to the orientation control layer 75 is conductive (metal) . From Figs. 8A and 8B, it can be seen that a voltage of 0.38 V is generated when the orientation control layer 75 has an insulation property and that a voltage of 0.01 V is generated when the orientation control layer 75 is conductive. Therefore, in the receiving element having the electrode arrangement of the present embodiment, it can be seen that it is essential for the orientation control layer 75 to have an insulation property in order to obtain high receiving sensitivity. If the orientation control layer 75 has an insulation property, electrical flux lines generated from the first electrode 61 are directly absorbed into the second electrode 62 through the piezoelectric body 63 (case 1). On the other hand, if the orientation control layer is conductive, electrical flux lines generated from the first electrode 61 once pass through the orientation control layer through the piezoelectric body 63, and then are absorbed into the second electrode 62 through the piezoelectric body 63 again (case 2) . This reason is shown below. Assuming that a charge generated by piezoelectricity is Q, the electrostatic capacitance of a capacitor forming the electrodes 61 and 62 is C, and a generated electric potential is V, the relationship of Q = C·V is generally satisfied. Since the value of the electrostatic capacitance C in the case 1 is about 1/30 times smaller than that in the case 2, the generated electric potential V is increased if the charge Q is fixed. The reason why the electrostatic capacitance C in the case 1 is small is that a distance, by which electrical flux lines travel through the piezoelectric body 63, is larger than in the case 2. In other words, the arrangement situation of the case 2 is expressed as "if a bottom surface in a gap between electrodes is in contact with a conductive material, a parasitic capacitance connecting the electrodes to each other is formed, and (total electrostatic capacitance is increased) receiving sensitivity is reduced". Here, as general material properties, a material having a resistance value of 1 × 10⁶ Qcm or more is believed to have an insulation property. If a material has a resistance value less than the resistance value of 1 × 10⁶ Ωcm, the material is a semiconductor, and is not suitable for the orientation control layer 75 according to the invention.

### 6 Reason why the piezoelectric layer should have (100) orientation rather than (001) orientation

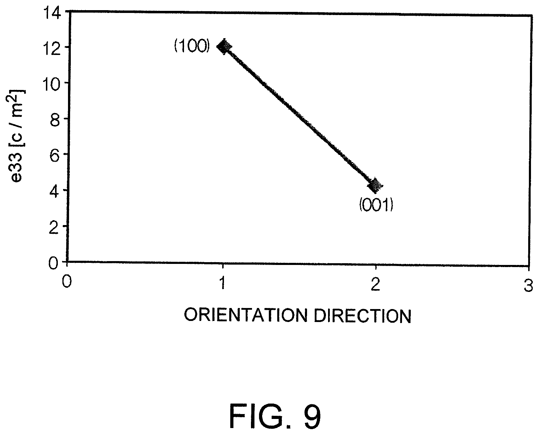

Electronic state calculation based on the first principle of solid shows that piezoelectricity is increased when the piezoelectric body 63 formed of PZT has (100) orientation rather than (001) orientation. The calculation is based on the density functional theory within local density approximation. A piezoelectric constant was calculated using a linear response theory. Energy cutoff was set to 500 eV, and the mesh of k-space was set to 3 × 3 × 3. A crystal structure for which the above calculation is to be performed is Pb(Zr0.5Ti0.5)O₃ having an ABO₃ perovskite structure. ABO₃ type structures corresponding to 2 x 2 × 2 = 8 cells were prepared as a super cell, and periodic boundary conditions were used. In the following explanation, a direction perpendicular to the plane is assumed to be a c axis, and directions perpendicular to each other within the plane are assumed to be a and b axes. As a result of the calculation, in the case of (001) orientation, the crystal lattice extends in the c-axis direction. The rate of extension in the c-axis direction is about c/a = 1.03. In this case, in the displacement of each atom from the central symmetry position, the <001> direction is a main displacement direction. On the other hand, in the case of (100) orientation, the crystal lattice satisfies the relationship of a = b = c. In this case, in the displacement of each atom from the central symmetry position, the <111> direction is a main displacement direction. Fig. 9 shows the relationship between a piezoelectric constant (e constant) and an orientation direction obtained by calculation. From Fig. 9, it can be seen that PZT gives a large piezoelectric constant at the time of (100) orientation rather than (001) orientation, which is advantageous.

### 7 Operation of the ultrasonic diagnostic apparatus

Next, the operation of the ultrasonic diagnostic apparatus 11 will be described briefly. In the transmission array TR, a pulse signal is supplied to the vibrator 25. The pulse signal is supplied to the second piezoelectric device 23 in each column through the lower electrode terminals 35 and 37 and the upper electrode terminals 34 and 36. In each second piezoelectric device 23, an electric field is applied to the piezoelectric body 28 between the lower electrode 27 and the upper electrode 26. The piezoelectric body 28 vibrates at a frequency of an ultrasonic wave. The vibration of the piezoelectric body 28 is transmitted to the vibrating film 24. Accordingly, the vibrating film 24 performs ultrasonic vibration. As a result, a desired ultrasonic beam is emitted toward a subject (for example, the inside of a human body).

In the receiving array RR, the supply of power to the first piezoelectric device 57 is switched for each group (a plurality of columns) interposed between the third and fourth conductors 67 and 68. The first piezoelectric device 57 receives an ultrasonic wave for each group of a plurality of columns. For each first piezoelectric device 57, the vibrating film 58 performs ultrasonic vibration. The ultrasonic vibration of the vibrating film 58 generates a strain of the piezoelectric body 63 at a desired frequency. The strain of the piezoelectric body 63 generates an electric potential. According to the piezoelectric effect of the vibrator 59, a voltage is output from the vibrator 59. Between the first and second electrodes 61 and 62, the (100) preferential orientation is established in the piezoelectric body 63. For this reason, a relatively large electric potential for the strain of the piezoelectric body 63 is generated. Such an electric potential is detected from the first and second electrodes. The electric potential is output as an electrical signal from the signal terminal 71 and the common terminal 72. In this manner, an ultrasonic wave is detected.

Transmission and reception of ultrasonic waves are repeated. As a result, a linear scan or a sector scan is realized. If the scan is completed, an image is formed based on the digital signal of the output signal. The formed image is displayed on the screen of the display panel 15.

In the first piezoelectric device 57, between the first and second electrodes 61 and 62, the bottom surface of the orientation region 74 is in contact with the insulating surface region 65. Accordingly, since the formation of a parasitic capacitance connecting the electrodes 61 and 62 to each other is avoided, good receiving sensitivity can be secured. If the bottom surface of the orientation region 74 is in contact with a conductive material, a parasitic capacitance connecting the electrodes to each other is formed. Therefore, the receiving sensitivity is lowered.

In order to obtain high receiving sensitivity, it is preferable that electrical flux lines generated when the piezoelectric body 63 is deformed by sound pressure extend directly to the second electrode 62 through the piezoelectric body 63 from the first electrode 61. For example, when viewed from above, if a region X including a conductive material is present in the vibrating film 58 below the orientation region 74, electrical flux lines extend to the second electrode 62 through the region X from the first electrode 61. In this case, the electrostatic capacitance C of a capacitor formed by the first and second electrodes 61 and 62 is effectively increased. As a result, the receiving sensitivity is lowered. As the distance between electrodes decreases, the electrostatic capacitance C increases. Since Q = CV, V is reduced when Q is fixed. This is not preferable.

### 8 Piezoelectric device according to a second embodiment

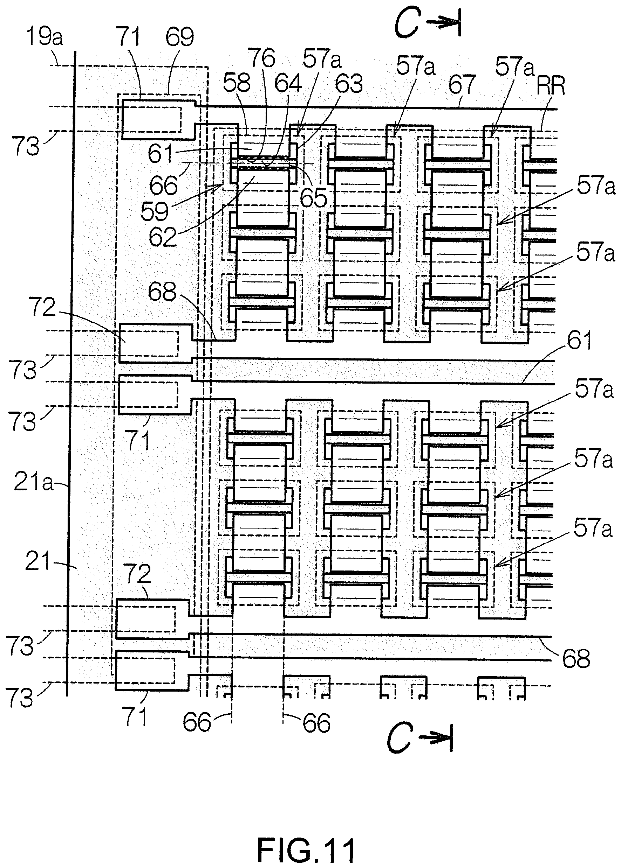

Fig. 11 shows the configuration of a first piezoelectric device 57a according to a second embodiment which is in accordance with the present invention. On the second surface 63b of the piezoelectric body 63, a groove 76 is formed between the first and second electrodes 61 and 62. The groove 76 passes through the gap 64 in a plan view along at least the reference line 66. Here, the groove 76 traverses the entire one surface from one edge to the other edge of the piezoelectric body 63 to bisect the second surface 63b of the piezoelectric body 63 instead of bisecting the second surface 63b of the piezoelectric body 63 in the gap 64.

In the first piezoelectric device 57a, as the distance between the first and second electrodes 61 and 62 increases, the electrostatic capacitance C of a capacitor formed by both the electrodes decreases. Accordingly, the generated voltage V of the piezoelectric body 63 is increased. In this case, as shown in Fig. 11, since distortion occurring in the piezoelectric body 63 in a region between electrodes is increased due to the groove 76, a large voltage is generated. In addition, in the first piezoelectric device 57a, a voltage is applied in parallel to the surface of the piezoelectric body 63. Therefore, compared with a case where a voltage is applied perpendicular to the surface, polarization sufficiently remains in the piezoelectric body 63. As a result, the application of a polarization voltage can be omitted (or reduced) at the time of generation of the piezoelectric effect.
In particular, the thickness of the piezoelectric body 63 is locally reduced in the groove 76. Accordingly, since the strain of the piezoelectric body 63 concentrates on the path of the electrical flux lines between the first and second electrodes 61 and 62, the piezoelectric effect can be efficiently used.

The groove 76 extends along the reference line 66 passing through the center of gravity of the vibrating film 58 in a plan view. In the vibrating film 58, bending at the time of ultrasonic vibration increases as a distance from the center-of-gravity position decreases. In this manner, if the groove 76 is disposed at a position where bending is likely to occur, the generated voltage is increased. In particular, the vibrating film 58 is formed in a rectangular shape in a plan view. The bending of the vibrating film 58 is maximized at an intermediate position equidistant from two sides extending in parallel to each other. If the groove 76 extends in parallel to the long side of the rectangle, the generated voltage is increased. Here, since the groove 76 traverses the entire one surface from one edge to the other edge of the piezoelectric body 63, the distortion of the piezoelectric body 63 is increased to the maximum extent. The generated voltage is increased to the maximum extent. The piezoelectric body 63 is formed symmetrically with respect to a straight line 65 in a plan view, and the behavior of the piezoelectric body 63 maintains the symmetry. Therefore, the behavior of the piezoelectric body 63 is stabilized at the time of vibration of the vibrating film 58.

### 9 Method of manufacturing a piezoelectric device according to the second embodiment

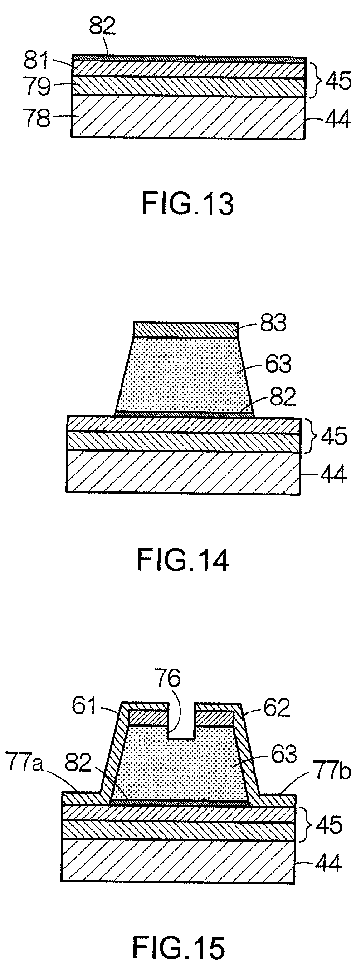

Next, a method of manufacturing the first piezoelectric device 57a will be described briefly. As shown in Fig. 13, a substrate 78 is prepared. The substrate 78 is formed of, for example, silicon. The silicon oxide layer 79 and the zirconium oxide layer 81 are formed on the surface of the substrate 78. In forming the silicon oxide layer 79, for example, heat treatment may be performed on the surface of the substrate 78. The silicon of the substrate 78 is oxidized to form a silicon oxide. In forming the zirconium oxide layer 81, a zirconium film is formed in a uniform thickness. Oxidation treatment is performed on the zirconium film. In this manner, the substrate 44 and the coat layer 45 are obtained.

A Bi (FeTi) O₃ layer 82 is formed on the surface of the coat layer 45. The Bi(FeTi)O₃ layer 82 corresponds to the orientation control layer 75 according to the invention. In forming the Bi(FeTi)O₃ layer 82, for example, a chemical solution method such as a metal-organic decomposition (MOD) method or a sol-gel method, a laser ablation method, a sputtering method, a pulsed laser deposition method (PLD method), a CVD method, and an aerosol deposition method can be used. In the MOD method, the Bi (FeTi) O₃ layer 82 is obtained by applying a metal complex solution containing Bi, Fe, and Ti, drying the metal complex solution, and baking the metal complex solution at high temperature. The Bi (FeTi) O₃ layer 82 establishes (100) preferential orientation on the randomly oriented zirconium oxide layer 81 regardless of the material of an underlying layer.

The thickness of the Bi (FeTi) O₃ layer 82 may be 3 nm to 100 nm. The obtained Bi(FeTi)O₃ layer 82 has an ABO₃ perovskite structure. Bi is located in the A site. Fe and Ti are located in the B site. The obtained Bi(FeTi)O₃ layer 82 has an insulation property. If the thickness of the Bi (FeTi) O₃ layer 82 is smaller than 3 nm, it is not possible to take a manufacturing margin. If the thickness of the Bi (FeTi) O₃ layer 82 exceeds 100 nm, a diaphragm is difficult to bend.

### 10 Manufacturing of the orientation control layer

As a specific example of a formation procedure in the case of forming the orientation control layer 75 using a chemical solution method, first, an orientation control layer precursor layer is formed by applying a composite for forming an orientation control layer (precursor solution of the orientation control layer), which is a MOD solution or a sol containing a metal complex containing Bi, Fe, and Ti, using a spin coat method or the like (orientation control layer precursor solution application step) . The precursor solution to be applied is obtained by mixing a metal complex capable of forming a composite oxide having a perovskite structure, in which Bi is included in the A site and Fe and Ti are included in the B site, by baking and then dissolving or dispersing the mixture in an organic solvent. As a metal complex containing Bi, Fe, or Ti, for example, alkoxides, organic acid salts, and β-diketone complexes can be used. As a metal complex containing Bi, for example, 2-ethyl hexane acid bismuth and bismuth acetate can be mentioned. As a metal complex containing Fe, for example, 2-ethyl hexane iron, iron acetate, and tris (acetylacetonate) iron can be mentioned. As a metal complex containing Ti, for example, 2-ethyl hexane acid titanium and titanium acetate can be mentioned. In addition, as solvents of the precursor solution of the orientation control layer, propanol, butanol, pentanol, hexanol, octanol, ethylene glycol, propylene glycol, octane, decane, cyclohexane, xylene, toluene, tetrahydrofuran, acetic acid, octyl acid, and the like can be mentioned. The composition ratio of Bi, Fe, and Ti is preferably Bi:Fe:Ti = 100:50:50.

Then, the orientation control layer precursor layer is heated to predetermined temperature (for example, 150°C to 200°C), and is dried for a certain period of time (orientation control layer drying step). Then, the dried orientation control layer precursor layer is heated to predetermined temperature (for example, 350°C to 450°C) and is held for a certain period of time so as to be degreased (orientation control layer degreasing step) . Here, "degreasing" means that organic components contained in the orientation control layer precursor layer are separated as NO₂, CO₂, H₂O, and the like. The atmosphere of the orientation control layer drying step or the orientation control layer degreasing step is not limited, and the orientation control layer drying step or the orientation control layer degreasing step may be performed in the air, or in an oxygen atmosphere, and in an inert gas atmosphere.

Then, the orientation control layer precursor layer is heated to predetermined temperature, for example, about 600°C to 850°C and is held for a certain period of time, for example, 1 minute to 10 minutes, so as to be crystallized. As a result, the orientation control layer 75 formed of a composite oxide having a perovskite structure, in which Bi is included in the A site and Fe and Ti are included in the B site, is obtained (baking step).

The atmosphere of the orientation control layer baking step is not limited either, and the orientation control layer baking step may be performed in the air, or in an oxygen atmosphere, and in an inert gas atmosphere. Examples of a heating device used in the orientation control layer drying step, the orientation control layer degreasing step, and the orientation control layer baking step include a rapid thermal annealing (RTA) device that performs heating by irradiation of an infrared lamp, a hot plate, and the like.

The piezoelectric body 63 is formed on the Bi (FeTi) O₃ layer 82 by the MOD method. The thickness of the piezoelectric body 63 may be 100 nm to 2000 nm. If the thickness of the piezoelectric body 63 is smaller than 100 nm, the influence of crystal defects, such as Pb or oxygen, is increased. Accordingly, since piezoelectricity is lowered, the receiving sensitivity is lowered. If the thickness of the piezoelectric body 63 is greater than 2000 nm, a crack is likely to occur in the piezoelectric body 63. This is not preferable in terms of manufacturing.

### 11 Manufacturing of the piezoelectric body

For example, first, a piezoelectric precursor layer is formed by applying a sol or a MOD solution (precursor solution), which contains an organic metal complex containing the constituent metals of a piezoelectric material for the piezoelectric body 63, on the orientation control layer 75 using a spin coat method or the like (application step) . The precursor solution to be applied is obtained, for example, by mixing an organic metal complex containing the constituent metals of a piezoelectric material for the piezoelectric body 63 so that the molar ratio between the constituent metals becomes a desired molar ratio and then dissolving or dispersing the mixture using an organic solvent, such as alcohol. As an organic metal complex containing the constituent metals of a piezoelectric material, for example, metal alkoxides, organic acid salts, and β-diketone complexes can be used. Specifically, for example, the following materials can be mentioned. As an organic metal complex containing lead (Pb), for example, lead acetate can be mentioned. As an organic metal complex containing zirconium (Zr), for example, zirconium acetylacetonate, zirconium tetra acetylacetonate, zirconium mono acetylacetonate, and zirconium bisacetylacetonate can be mentioned. As an organic metal complex containing titanium (Ti), for example, titanium alkoxide and titanium isopropoxide can be mentioned. The composition ratio of Pb, Zr, and Ti, is preferably Pb:Zr:Ti = 120:52:48.

Then, the piezoelectric precursor layer is heated to predetermined temperature, for example, about 130°C to 180°C, and is dried for a certain period of time (drying step) . Then, the dried piezoelectric precursor layer is heated to predetermined temperature, for example, 300°C to 400°C and is held for a certain period of time so as to be degreased (degreasing step). Here, "degreasing" means that organic components contained in the piezoelectric precursor layer are separated as NO₂, CO₂, H₂O, and the like.

Then, the dried piezoelectric precursor layer is heated to predetermined temperature, for example, 650°C to 800°C and is held for a certain period of time so as to be crystallized. As a result, a piezoelectric layer is formed (baking step) . Examples of a heating device used in the drying step, the degreasing step, and the baking step include a rapid thermal annealing (RTA) device that performs heating by irradiation of an infrared lamp, a hot plate, and the like. In addition, the piezoelectric body 63 configured to include a plurality of piezoelectric layers may be formed by repeating the application step, the drying step, and the degreasing step described above or the application step, the drying step, the degreasing step, and the baking step described above according to desired thickness or the like.

Then, the piezoelectric body 63 with a predetermined thickness that is configured to include a plurality of piezoelectric layers is formed by repeating the application step, the drying step, and the degreasing step described above or the application step, the drying step, the degreasing step, and the baking step described above according to desired thickness or the like. For example, in a case where the film thickness of the coating solution per time is about 0.1 µm, the total thickness of the piezoelectric body 63 configured to include, for example, ten piezoelectric layers is about 1.1 µm. Although the piezoelectric body 63 is provided by laminating the piezoelectric layers in the present embodiment, the piezoelectric body 63 may be formed to have only one piezoelectric layer.

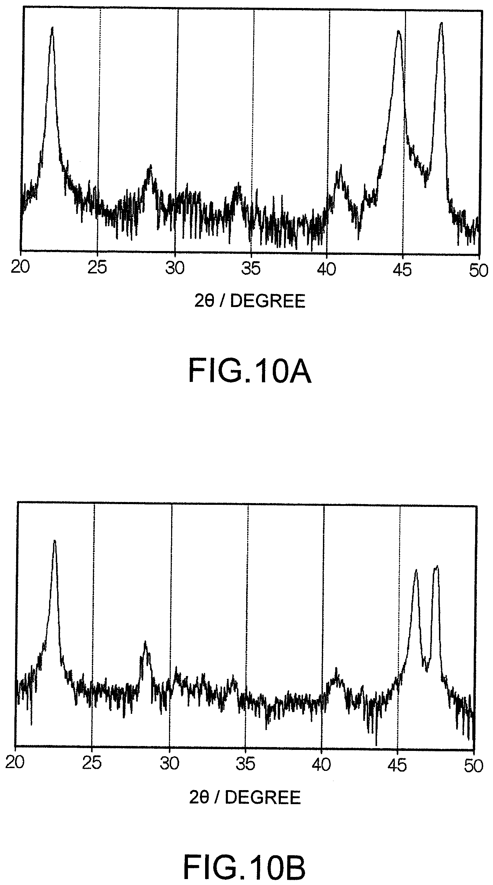

Figs. 10A and 10B are X-ray diffraction patterns showing a state in which the piezoelectric body 63 is (100) preferentially oriented in a case where the piezoelectric body 63 is formed on the orientation control layer 75. The horizontal axis indicates 2θ, and the vertical axis indicates an X-ray diffraction intensity. Kα-rays of Cu were used as X-rays. Fig. 10A shows a case in which the piezoelectric body 63 is PZT, and Fig. 10B shows a case in which the piezoelectric body 63 is BFM-BT. Here, PZT has a composition of Zr:Ti of 52:48. In addition, BFM-BT is an abbreviation of (BiFeMnO₃)_{0.7} - (BaTiO₃)_{0.3}. There is a main diffraction peak in the range of 2θ = 21° to 24° on the 2θ axis, and diffraction peaks from other orientations, such as (111) or (110), are not observed in other angular regions. Therefore, it can be said that the piezoelectric body 63 having a perovskite structure is completely (100) preferentially oriented. In the present embodiment, there is a diffraction peak at 21.88° in the case of PZT and 22. 54° in the case of BFM-BT. Therefore, in all cases, it was confirmed that the piezoelectric body 63 was (100) preferentially oriented.

As shown in Fig. 14, the piezoelectric body 63 and an underlying conductive layer 83 are formed on the surface of the coat layer 45. The underlying conductive layer 83 is formed by sputtering. The underlying conductive layer 83 is patterned on a piezoelectric material layer of a solid layer. In the piezoelectric material layer, (100) preferential orientation is established by the underlying Bi (FeTi) O₃ layer 82. Then, etching processing is performed on the piezoelectric material layer. By the piezoelectric material layer, the piezoelectric body 63 is formed. The underlying conductive layer 83 is laminated on the top surface of the piezoelectric body 63.

As shown in Fig. 15, an electrode layer of a solid layer is formed on the underlying conductive layer 83 by sputtering. The electrode layer is etched according to predetermined patterning. In this manner, the first and second electrodes 61 and 62 are formed on the piezoelectric body 63. The electrode layer forms the lead-out wiring lines 77a and 77b that are separately connected to the first and second electrodes 61 and 62. At this time, on the top surface of the piezoelectric body 63, the groove 76 is formed between the first and second electrodes 61 and 62 according to over-etching. According to the formation of the groove 76, the first and second electrodes 61 and 62 are separated from each other. Then, on the substrate 78, the opening 46 is formed from the back surface.

It is preferable that a gap distance L between the first and second electrodes 61 and 62 is 2 µm or more. As the gap distance L increases, the electrostatic capacitance C of a capacitor decreases. Accordingly, the output voltage V is increased. On the other hand, if the gap distance L is too large, the initializing voltage of the piezoelectric body 63 is increased to 100 V or more. If the initializing voltage is too large, the cost of a driver IC is increased. Therefore, it is preferable that the gap distance L is 2 µm or more and 8 µm or less.

### 12 Measurement of receiving sensitivity

The measurement procedure of receiving sensitivity is shown below. A receiving element obtained through the above process is placed in a water tank filled with water, and an ultrasonic wave is generated from a large-diameter hydrophone located 30 cm away from the water tank. At this time, a voltage generated between the first and second electrodes 61 and 62 of the receiving element is read using a voltage amplifier. A peak-to-peak output voltage per 1 Pa is expressed as an indicator of receiving sensitivity. As an ultrasonic wave generated by the large-diameter hydrophone, a pulse wave of the 1.5 wave is generated with 3.5 MHz as a fundamental wave. Prior to reception measurement, in order to initialize the polarization of the piezoelectric body 63, a voltage of 100 V is applied between the first and second electrodes 61 and 62 of the receiving element.

One form of the embodiment is shown below. The vibrating film 58 and the piezoelectric body 63 are squares when viewed from above. The length of one side of a diaphragm formed by the vibrating film 58 is 40 µm, and the length of one side of the piezoelectric body 63 is 32 µm. In addition, the vibrating film 58 includes the silicon oxide layer 48 (1000 nm), the zirconium oxide layer 49 (400 nm), BFT (20 nm) of the orientation control layer 75, and PZT (1350 nm) of the piezoelectric body 63 in order from below. BFT is the abbreviation of BiFeTiO₃ that forms the orientation control layer 75 according to the invention. The size of the gap 64 between the first and second electrodes 61 and 62 is set to 5 µm. In this case, the resonance frequency of the diaphragm in water is about 7.5 MHz.

In the case of using the orientation control layer 75 according to the invention, PZT of the piezoelectric body 63 is (100) preferentially oriented. On the other hand, as a comparative example, in a case where no orientation control layer is used, PZT of the piezoelectric body 63 is randomly oriented. The receiving sensitivity in the case of using the orientation control layer according to the invention was 3000 nV/Pa. On the other hand, in a case where no orientation control layer was used as in the comparative example, the receiving sensitivity was 2000 nV/Pa. Therefore, it can be seen that the invention using the orientation control layer has higher receiving sensitivity than in the comparative example.

While the second embodiment has been described in detail above, it could be easily understood by those skilled in the art that various changes and modifications thereof could be made without departing from the scope of the invention as defined by the claims. Accordingly, all of such modification examples are still included in the range of the invention. For example, in the specification or diagrams, a term which is described at least once together with different terms having a broader meaning or the same meaning can be replaced with the different terms in any parts of the specification or diagrams. In addition, the configurations and operations of the ultrasonic device unit 17, the device terminal 12, the second piezoelectric device 23, the coat layer 45, the vibrator 59, and the like are not limited to those described in the second embodiment, and various modifications can be made.

## Claims

1. A piezoelectric device (57), comprising:
an elastic layer (58) that forms an insulating surface region at least partially and has an amorphous structure or random orientation at least in the surface region;
a piezoelectric body (63) that is provided on the elastic layer (58), and has a first surface (63a) in contact with the elastic layer (58) and a second surface (63b) on an opposite side to the first surface;
a first electrode (61) electrically connected to the piezoelectric body (63); and
a second electrode (62) electrically connected to the piezoelectric body (63),
**characterized in that**
the piezoelectric body (63) is (1 0 0) preferentially oriented in an orientation region corresponding to the surface region in a plan view,
wherein a gap (64) is formed between the first (61) and second electrodes (62) corresponding to the orientation region in the plan view, and
a groove (76) is formed on the second surface (63b) of the piezoelectric body (63) in the gap (64).

2. The piezoelectric device (57) according to claim 1, wherein the piezoelectric body (63) is formed of a transition metal oxide having a perovskite structure.

3. The piezoelectric device (57) according to claim 1 or 2, wherein the first surface (63a) of the piezoelectric body (63) is formed as an orientation control layer that is formed of a transition metal oxide having a perovskite structure.

4. The piezoelectric device (57) according to claim 3, wherein the orientation control layer is formed of a transition metal oxide having a perovskite structure that contains bismuth in an A site and iron and titanium in a B site.

5. The piezoelectric device (57) according to claim 3 or 4, wherein a resistance value of the orientation control layer is equal to or greater than 10⁶ Ωcm.

6. The piezoelectric device (57) according to any one of claims 3 to 5, wherein the orientation control layer is (1 0 0) preferentially oriented.

7. The piezoelectric device (57) according to any one of claims 3 to 6, wherein the piezoelectric body (63) includes a Pb(ZrTi)O₃ layer laminated on the orientation control layer.

8. The piezoelectric device (57) according to any one of claims 3 to 7, wherein the orientation control layer contains Pb.

9. The piezoelectric device (57) according to any one of claims 3 to 8, wherein the orientation control layer is a continuous layer.

10. The piezoelectric device (57) according to any one of claims 3 to 8, wherein the orientation control layer is a discontinuous layer.

11. A probe (13), comprising:
the piezoelectric device (57) according to any one of claims 1 to 10; and
a housing (16) that supports the piezoelectric device (57) .

12. An electronic apparatus, comprising:
the piezoelectric device (57) according to any one of claims 1 to 10; and
a processing circuit that is connected to the piezoelectric device (57) and processes an output of the piezoelectric device (57).

13. An ultrasonic imaging apparatus (11), comprising:
the piezoelectric device (57) according to any one of claims 1 to 10;
a processing circuit that is connected to the piezoelectric device (57) and processes an output of the piezoelectric device (57) to generate an image; and
a display device that displays the image.

## Patentansprüche

1. Piezoelektrische Vorrichtung (57), umfassend:
eine elastische Schicht (58), die wenigstens teilweise eine isolierende Oberflächenregion bildet und wenigstens in der Oberflächenregion eine amorphe Struktur oder eine zufällige Orientierung aufweist;
einen piezoelektrischen Körper (63), der auf der elastischen Schicht (58) bereitgestellt ist und eine erste Oberfläche (63a) in Kontakt mit der elastischen Schicht (58) und eine zweite Oberfläche (63b) auf einer gegenüberliegenden Seite zur ersten Oberfläche aufweist;
eine erste Elektrode (61), die mit dem piezoelektrischen Körper (63) elektrisch verbunden ist; und
eine zweite Elektrode (62), die mit dem piezoelektrischen Körper (63) elektrisch verbunden ist,
**dadurch gekennzeichnet, dass**
der piezoelektrische Körper (63) in einer Orientierungsregion, die in einer Draufsicht der Oberflächenregion entspricht, vorzugsweise (100) orientiert ist,
wobei ein Spalt (64) zwischen der ersten (61) und der zweiten Elektrode (62) gebildet ist, der in der Draufsicht der Orientierungsregion entspricht, und
eine Nut (76) auf der zweiten Oberfläche (63b) des piezoelektrischen Körpers (63) im Spalt (64) gebildet ist.

2. Piezoelektrische Vorrichtung (57) nach Anspruch 1, wobei der piezoelektrische Körper (63) aus einem Übergangsmetalloxid mit einer Perovskit-Struktur gebildet ist.

3. Piezoelektrische Vorrichtung (57) nach Anspruch 1 oder 2, wobei die erste Oberfläche (63a) des piezoelektrischen Körpers (63) als eine Orientierungssteuerungsschicht gebildet ist, die aus einem Übergangsmetalloxid mit einer Perovskit-Struktur gebildet ist.

4. Piezoelektrische Vorrichtung (57) nach Anspruch 3, wobei die Orientierungssteuerungsschicht aus einem Übergangsmetalloxid mit einer Perovskit-Struktur gebildet ist, die Bismut an einer A-Stelle und Eisen und Titan an einer B-Stelle enthält.

5. Piezoelektrische Vorrichtung (57) nach Anspruch 3 oder 4, wobei ein Widerstandswert der Orientierungsteuerungsschicht gleich oder größer als 10⁶ Ωcm ist.

6. Piezoelektrische Vorrichtung (57) nach einem der Ansprüche 3 bis 5, wobei die Orientierungssteuerungsschicht vorzugsweise (100) orientiert ist.

7. Piezoelektrische Vorrichtung (57) nach einem der Ansprüche 3 bis 6, wobei der piezoelektrische Körper (63) eine Pb(ZrTi)O₃-Schicht einschließt, die auf die Orientierungssteuerungsschicht laminiert ist.

8. Piezoelektrische Vorrichtung (57) nach einem der Ansprüche 3 bis 7, wobei die Orientierungssteuerungsschicht Pb enthält.

9. Piezoelektrische Vorrichtung (57) nach einem der Ansprüche 3 bis 8, wobei die Orientierungssteuerungsschicht eine kontinuierliche Schicht ist.

10. Piezoelektrische Vorrichtung (57) nach einem der Ansprüche 3 bis 8, wobei die Orientierungssteuerungsschicht eine diskontinuierliche Schicht ist.

11. Sonde (13), umfassend:
die piezoelektrische Vorrichtung (57) nach einem der Ansprüche 1 bis 10; und
ein Gehäuse (16), das de piezoelektrische Vorrichtung (57) trägt.

12. Elektronische Vorrichtung, umfassend:
die piezoelektrische Vorrichtung (57) nach einem der Ansprüche 1 bis 10; und
eine Verarbeitungsschaltung, die mit der piezoelektrischen Vorrichtung (57) verbunden ist und eine Ausgabe der piezoelektrischen Vorrichtung (57) verarbeitet.

13. Ultraschallabbildungsvorrichtung (11), umfassend:
die piezoelektrische Vorrichtung (57) nach einem der Ansprüche 1 bis 10;
eine Verarbeitungsschaltung, die mit der piezoelektrischen Vorrichtung (57) verbunden ist und eine Ausgabe der piezoelektrischen Vorrichtung (57) verarbeitet, um ein Bild zu erzeugen; und
eine Anzeigevorrichtung, die das Bild anzeigt.

## Revendications

1. Dispositif piézoélectrique (57) comprenant :
une couche élastique (58) qui forme une région de surface isolante au moins partiellement et qui présente une structure amorphe ou une orientation aléatoire au moins dans la région de surface ;
un corps piézoélectrique (63) qui est ménagé sur la couche élastique (58) et qui a une première surface (63a) en contact avec la couche élastique (58) et une seconde surface (63b) sur un côté opposé à la première surface ;
une première électrode (61) reliée électriquement au corps piézoélectrique (63); et
une seconde électrode (62) reliée électriquement au corps piézoélectrique (63) ;
**caractérisé en ce que**
le corps piézoélectrique (63) est orienté préférentiellement (1 0 0) dans une région d'orientation correspondant à la région de surface dans une vue en plan ;
dans lequel un espace (64) est formé entre les première (61) et seconde électrodes (62) correspondant à la région d'orientation dans la vue en plan ; et
une rainure (76) est formée sur la seconde surface (63b) du corps piézoélectrique (63) dans l'espace (64).

2. Dispositif piézoélectrique (57) selon la revendication 1, dans lequel le corps piézoélectrique (63) est formé d'un oxyde de métal de transition ayant une structure pérovskite.

3. Dispositif piézoélectrique (57) selon la revendication 1 ou 2, dans lequel la première surface (63a) du corps piézoélectrique (63) est formée comme une couche de commande d'orientation qui est formée d'un oxyde de métal de transition ayant une structure pérovskite.

4. Dispositif piézoélectrique (57) selon la revendication 3, dans lequel la couche de commande d'orientation est formée d'un oxyde de métal de transition ayant une structure pérovskite qui contient du bismuth dans un site A et du fer et du titane dans un site B.

5. Dispositif piézoélectrique (57) selon la revendication 3 ou 4, dans lequel une valeur de résistance de la couche de commande d'orientation est égale ou supérieure à 10⁶ Ωcm.

6. Dispositif piézoélectrique (57) selon l'une quelconque des revendications 3 à 5, dans lequel la couche de commande d'orientation est orientée préférentiellement (1 0 0).

7. Dispositif piézoélectrique (57) selon l'une quelconque des revendications 3 à 6, dans lequel le corps piézoélectrique (63) inclut une couche de Pb(ZrTi)O₃ stratifiée sur la couche de commande d'orientation.

8. Dispositif piézoélectrique (57) selon l'une quelconque des revendications 3 à 7, dans lequel la couche de commande d'orientation contient du Pb.

9. Dispositif piézoélectrique (57) selon l'une quelconque des revendications 3 à 8, dans lequel la couche de commande d'orientation est une couche continue.

10. Dispositif piézoélectrique (57) selon l'une quelconque des revendications 3 à 8, dans lequel la couche de commande d'orientation est une couche discontinue.

11. Sonde (13) comprenant :
le dispositif piézoélectrique (57) selon l'une quelconque des revendications 1 à 10; et
un boîtier (16) qui sert de support au dispositif piézoélectrique (57).

12. Appareil électronique comprenant :
le dispositif piézoélectrique (57) selon l'une quelconque des revendications 1 à 10; et
un circuit de traitement qui est relié au dispositif piézoélectrique (57) et traite une sortie du dispositif piézoélectrique (57).

13. Appareil d'imagerie par ultrasons (11) comprenant :
le dispositif piézoélectrique (57) selon l'une quelconque des revendications 1 à 10;
un circuit de traitement qui est relié au dispositif piézoélectrique (57) et qui traite une sortie du dispositif piézoélectrique (57) pour générer une image ; et
un dispositif d'affichage qui affiche l'image.
